(19) **Europäisches Patentamt**
**European Patent Office**
**Office européen des brevets**

(11) **EP 4 567 679 A1**

(12) **EUROPEAN PATENT APPLICATION**
published in accordance with Art. 153(4) EPC

(43) Date of publication:
**11.06.2025 Bulletin 2025/24**

(21) Application number: **23872962.8**

(22) Date of filing: **21.09.2023**

(51) International Patent Classification (IPC):
**G06N 3/08** (2023.01)    **G06N 3/04** (2023.01)

(52) Cooperative Patent Classification (CPC):
**G06N 3/04; G06N 3/08**

(86) International application number:
**PCT/KR2023/014431**

(87) International publication number:
**WO 2024/071845 (04.04.2024 Gazette 2024/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.09.2022 KR 20220123712**

(71) Applicant: **Medical AI Co., Ltd.**
**Seoul 06180 (KR)**

(72) Inventors:
• **KWON, Joonmyoung**
**Seoul 06180 (KR)**
• **LEE, Byeongtak**
**Seoul 06180 (KR)**

(74) Representative: **Patentanwaltskanzlei Matschnig & Forsthuber OG**
**Biberstraße 22**
**Postfach 36**
**1010 Wien (AT)**

(54) **METHOD, PROGRAM, AND DEVICE FOR CONSTRUCTING MEDICAL ARTIFICIAL INTELLIGENCE MODEL**

(57) According to one embodiment of the present disclosure, there are disclosed a method, program and device for constructing a medical artificial intelligence model, which are performed by a computing device. The method may include: establishing evaluation criteria for an artificial intelligence model based on a task intended by a user; determining a first indicator used for loss computation for training an artificial intelligence model and a second indicator used for evaluation computation for selecting the trained model according to the established evaluation criteria; and constructing an artificial intelligence model that performs the task intended by the user based on the determined first and second indicators.

FIG. 2

## Description

### Technical Field

The present disclosure relates to deep learning technology in the medical field, and more particularly, to a method of constructing a medical artificial intelligence model that is suitable for a user's purpose.

### Background Art

In order to use an artificial intelligence model, it is important to train the artificial intelligence model desirably based on high-quality data that is suitable for a domain of use. However, training an artificial intelligence model with high-quality data does not guarantee that the artificial intelligence model will achieve performance that is suitable for a user's purpose and a domain of use. Accordingly, it is important to appropriately evaluate whether a trained model achieves desirable performance. In other words, evaluating a training model may ensure the reliability, efficiency and transparency of the model, and may prompt the continuous improvement of the model according to a user's purpose and a domain of use.

The criteria for evaluating a trained model are generally set independently of training conditions and parameters for the model. In other words, evaluation is performed on a trained model in the process of constructing an artificial intelligence model, so that evaluation criteria themselves do not directly affect the determination of training conditions or parameters for the model. Furthermore, the criteria for evaluating a trained model may include one or more evaluation indicators specific to the purpose or domain of use in addition to standard performance indicators, so that it is difficult to set the training conditions and parameters for the model by reflecting therein the criteria that can change flexibly.

However, as an artificial intelligence model achieves performance suitable for the evaluation criteria better, a model that performs the ultimate task that the artificial intelligence model attempts to implement can be developed better. Therefore, in the cases where it is necessary to include one or more evaluation indicators specific to the purpose or domain of use, as in the medical field, it can be seen that it is necessary to appropriately reflect therein the evaluation indicators specific to the purpose or domain of use throughout the overall process of developing the model.

### Disclosure

### Technical Problem

An object of the present disclosure is to provide a method of constructing a medical artificial intelligence model by deriving evaluation criteria for defining an artificial intelligence model based on a task intended by a user and configuring parameters for training and selecting the model to meet the evaluation criteria.

However, the objects to be accomplished by the present disclosure are not limited to the object mentioned above, and other objects not mentioned may be clearly understood based on the following description.

### Technical Solution

According to one embodiment of the present disclosure for achieving the above-described object, there is disclosed a method of constructing a medical artificial intelligence model. The method may include: establishing evaluation criteria for an artificial intelligence model based on a task intended by a user; determining a first indicator used for loss computation for training an artificial intelligence model and a second indicator used for evaluation computation for selecting the trained model according to the established evaluation criteria; and constructing an artificial intelligence model that performs the task intended by the user based on the determined first and second indicators.

Alternatively, the evaluation criteria may include at least one of: a first criterion for the accuracy of the artificial intelligence model; a second criterion for the uncertainty of the output of the artificial intelligence model; and a third criterion for the correlation between the output of the artificial intelligence model and a biometric value that determines whether a disease included in the task intended by the user has occurred.

Alternatively, when the task intended by the user is a prediction of left ventricular systolic dysfunction (LVSD), the biometric value that determines whether the disease has occurred may be a left ventricular ejection fraction (EF).

Alternatively, establishing the evaluation criteria for the artificial intelligence model based on the task intended by the user may include determining the ratio among the first criterion, the second criterion, and the third criterion in the evaluation criteria based on the task intended by the user.

Alternatively, when the task intended by the user is a prediction of left ventricular systolic dysfunction (LVSD), the ratio among the first criterion, the second criterion, and the third criterion in the evaluation criteria may be determined to be 4:3:3.

Alternatively, determining the first indicator used for the loss computation for training the artificial intelligence model and the second indicator used for the evaluation computation for selecting the trained model according to the established evaluation criteria may include determining a loss function included in the first indicator so that the correlation according to the third criterion can be calculated.

Alternatively, when the task intended by the user is a prediction of left ventricular systolic dysfunction (LVSD), the loss function included in the first indicator may include a left ventricular ejection fraction (EF) regression loss function.

Alternatively, determining the first indicator used for the loss computation for training the artificial intelligence model and the second indicator used for the evaluation computation for selecting the trained model according to the established evaluation criteria may include determining detailed indicators included in the second indicator according to the ratio among the first criterion, the second criterion, and the third criterion in the evaluation criteria.

Alternatively, establishing the evaluation criteria for the artificial intelligence model based on the task intended by the user may include: obtaining information about the task intended by the user based on user input; and deriving the evaluation criteria by inputting the information about the task intended by the user to a pre-trained criteria setting model.

Alternatively, establishing the evaluation criteria for the artificial intelligence model based on the task intended by the user may include: obtaining information about the task intended by the user based on user input; and identifying evaluation classifications and detailed criteria corresponding to the information about the task intended by the user from a preset database.

Alternatively, constructing the artificial intelligence model that performs the task intended by the user based on the determined first and second indicators may include: training the artificial intelligence model using the determined first indicator; evaluating the performance of the artificial intelligence model using the determined second indicator; and, when the evaluated performance of the artificial intelligence model satisfies the evaluation criteria, selecting an artificial intelligence model satisfying the evaluation criteria as the artificial intelligence model that performs the task intended by the user.

According to one embodiment of the present disclosure for achieving the above-described object, there is disclosed a computer program stored in a computer-readable storage medium. The computer program performs operations for constructing a medical artificial intelligence model when executed on one or more processors. The operations may include the operations of: establishing evaluation criteria for an artificial intelligence model based on a task intended by a user; determining a first indicator used for loss computation for training an artificial intelligence model and a second indicator used for evaluation computation for selecting the trained model according to the established evaluation criteria; and constructing an artificial intelligence model that performs the task intended by the user based on the determined first and second indicators.

According to one embodiment of the present disclosure for achieving the above-described object, there is disclosed a computing device for constructing a medical artificial intelligence model. The computing device may include: a processor including at least one core; memory including program codes executable on the processor; and a network unit for performing obtainment. In this case, the processor may establish evaluation criteria for an artificial intelligence model based on a task intended by a user, may determine a first indicator used for loss computation for training an artificial intelligence model and a second indicator used for evaluation computation for selecting the trained model according to the established evaluation criteria, and may construct an artificial intelligence model that performs the task intended by the user based on the determined first and second indicators.

**Advantageous Effects**

The present disclosure may provide an environment in which a model capable of achieving optimal performance suitable for the purpose of use and a task can be stably developed by allowing evaluation criteria to be reflected in the training and selection of the model.

**Description of Drawings**

FIG. 1 is a block diagram of a computing device according to one embodiment of the present disclosure;
FIG. 2 is a block diagram showing an operation process for constructing a medical artificial intelligence model according to one embodiment of the present disclosure; and
FIG. 3 is a flowchart showing a method of constructing a medical artificial intelligence model according to one embodiment of the present disclosure.

**Mode for Invention**

Embodiments of the present disclosure will be described in detail below with reference to the accompanying drawings so that those having ordinary skill in the art of the present disclosure (hereinafter referred to as those skilled in the art) can easily implement the present disclosure. The embodiments presented in the present disclosure are provided to enable those skilled in the art to use or practice the content of the present disclosure. Accordingly, various modifications to embodiments of the present disclosure will be apparent to those skilled in the art. That is, the present disclosure may be implemented in various different forms and is not limited to the following embodiments.

The same or similar reference numerals denote the same or similar components throughout the specification of the present disclosure. Additionally, in order to clearly describe the present disclosure, reference numerals for parts that are not related to the description of the present disclosure may be omitted in the drawings.

The term "or" used herein is intended not to mean an exclusive "or" but to mean an inclusive "or." That is, unless otherwise specified herein or the meaning is not clear from the context, the clause "X uses A or B" should be understood to mean one of the natural inclusive substitutions. For example, unless otherwise specified herein or the meaning is not clear from the context, the clause "X uses A or B" may be interpreted as any one of a case where X uses A, a case where X uses B, and a case where X uses both A and B.

The term "and/or" used herein should be understood to refer to and include all possible combinations of one or more of listed related concepts.

The terms "include" and/or "including" used herein should be understood to mean that specific features and/or components are present. However, the terms "include" and/or "including" should be understood as not excluding the presence or addition of one or more other features, one or more other components, and/or combinations thereof.

Unless otherwise specified herein or unless the context clearly indicates a singular form, the singular form should generally be construed to include "one or more."

The term "N-th (N is a natural number)" used herein may be understood as an expression used to distinguish the components of the present disclosure according to a predetermined criterion such as a functional perspective, a structural perspective, or the convenience of description. For example, in the present disclosure, components performing different functional roles may be distinguished as a first component or a second component. However, components that are substantially the same within the technical spirit of the present disclosure but should be distinguished for the convenience of description may also be distinguished as a first component or a second component.

The term "obtaining" used herein may be understood to mean not only receiving data over a wired/wireless communication network connecting with an external device or a system, but also generating data in an on-device form.

Meanwhile, the term "module" or "unit" used herein may be understood as a term referring to an independent functional unit processing computing resources, such as a computer-related entity, firmware, software or part thereof, hardware or part thereof, or a combination of software and hardware. In this case, the "module" or "unit" may be a unit composed of a single component, or may be a unit expressed as a combination or set of multiple components. For example, in the narrow sense, the term "module" or "unit" may refer to a hardware component or set of components of a computing device, an application program performing a specific function of software, a procedure implemented through the execution of software, a set of instructions for the execution of a program, or the like. Additionally, in the broad sense, the term "module" or "unit" may refer to a computing device itself constituting part of a system, an application running on the computing device, or the like. However, the above-described concepts are only examples, and the concept of "module" or "unit" may be defined in various manners within a range understandable to those skilled in the art based on the content of the present disclosure.

The term "model" used herein may be understood as a system implemented using mathematical concepts and language to solve a specific problem, a set of software units intended to solve a specific problem, or an abstract model for a process intended to solve a specific problem. For example, a neural network "model" may refer to an overall system implemented as a neural network that is provided with problem-solving capabilities through training. In this case, the neural network may be provided with problem-solving capabilities by optimizing parameters connecting nodes or neurons through training. The neural network "model" may include a single neural network, or a neural network set in which multiple neural networks are combined together.

The foregoing descriptions of the terms are intended to help to understand the present disclosure. Accordingly, it should be noted that unless the above-described terms are explicitly described as limiting the content of the present disclosure, they are not used in the sense of limiting the technical spirit of the present disclosure.

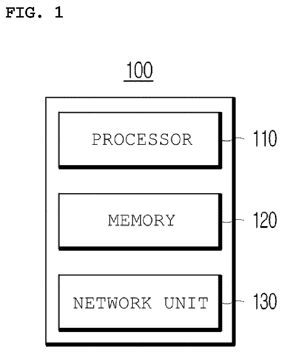

FIG. 1 is a block diagram of a computing device according to one embodiment of the present disclosure.

The computing device 100 according to the one embodiment of the present disclosure may be a hardware device or a part of a hardware device that performs the comprehensive processing and operation of data, or may be a software-based computing environment that is connected to a communication network. For example, the computing device 100 may be a server that is a principal agent performing an intensive data processing function and sharing resources, or may be a client that shares resources through interaction with a server or a specific terminal. Furthermore, the computing device 100 may be a cloud system in which pluralities of servers and clients comprehensively process data while interacting with each other. Furthermore, the computing device 100 may be a component of a medical robot. Since the above-described description is only one example related to the type of the computing device 100, the type of the computing device 100 may be configured in various manners within a range understandable to those skilled in the art based on the content of the present disclosure.

Referring to FIG. 1, the computing device 100 according to one embodiment of the present disclosure may include a processor 110, memory 120, and a network unit 130. However, FIG. 1 shows only an example, and the computing device 100 may include other components for implementing a computing environment. Furthermore, only some of the components disclosed above may be included in the computing device 100.

The processor 110 according to one embodiment of the present disclosure may be understood as a constituent unit including hardware and/or software for performing computing operation. For example, the processor 110 may read a computer program and perform data processing. The processor 110 may process operation processes such as the processing of input data for machine learning, the extraction of features for machine learning, and the computation of errors based on backpropagation. The processor 110 for performing such data processing may include a central processing unit (CPU), a general purpose graphics processing unit (GPGPU), a tensor processing unit (TPU), an application specific integrated circuit (ASIC), or a field programmable gate array (FPGA). Since the types of processor 110 described above are only examples, the type of processor 110 may be configured in various manners within a range understandable to those skilled in the art based on the content of the present disclosure.

The processor 110 may establish evaluation criteria based on information about a task intended by a user. The processor 110 may generate evaluation criteria based on information about a task obtained based on user input. For example, the processor 110 may generate a user interface for receiving information about a task intended by a user. In this case, the information input via the user interface may be basic information necessary for establishing evaluation criteria, such as the type of disease, the type of task and/or the like, or may be evaluation criteria themselves. The processor 110 may derive evaluation criteria by analyzing the basic information included in user input based on a pre-set database or a pre-trained criteria setting model. In this case, the database may be a structured dataset in which evaluation classifications such as accuracy and detailed criteria such as the area under ROC (AUROC) are matched for each task. Furthermore, the criteria setting model may be a neural network model pre-trained to receive information about a task intended by a user and derive evaluation classifications and detailed criteria. Although the criteria setting model may be trained based on supervised learning, it may also be trained based on unsupervised learning or self-supervised learning depending on the neural network structure or parameter configuration. The processor 110 may also obtain evaluation criteria themselves from user input and generate evaluation criteria.

The processor 110 may determine the indicators used for training and selecting an artificial intelligence model based on the established evaluation criteria. The processor 110 may generate training and selection indicators for the model that can reflect the evaluation criteria based on user input. The processor 110 may derive training and evaluation indicators corresponding to evaluation criteria by analyzing the evaluation criteria. For example, when the evaluation criteria are generated, the processor 110 may generate a user interface that lists the indicators that need to be constructed in an operation for the training or selection of the model to reflect the evaluation criteria therein. When user input for selecting indicators is obtained via the user interface, the processor 110 may select indicators according to the user input and determine training and evaluation indicators. When the evaluation criteria are generated, the processor 110 may generate training and selection indicators for the model by analyzing the evaluation criteria themselves without user input. The processor 110 may determine training and selection indicators for the model according to the evaluation classifications and detailed criteria included in the evaluation criteria based on a preset database or a pre-trained indicator determination model. In this case, the database may be a dataset in which training and selection indicators are structured along with evaluation classifications such as accuracy and detailed criteria such as AUROC (area under ROC) for each task. Furthermore, the indicator determination model may be a neural network model pre-trained to receive evaluation classifications and detailed criteria and derive training and selection indicators. Although the indicator determination model may be trained based on supervised learning, it may also be trained based on unsupervised learning or self-supervised learning depending on the neural network structure or parameter configuration.

The processor 110 may construct an artificial intelligence model using the training and selection indicators derived based on the evaluation criteria. The processor 110 may train the artificial intelligence model using the training indicators derived based on the evaluation criteria. Furthermore, the processor 110 may evaluate the performance of the trained artificial intelligence model using the selection indicators, derived based on the evaluation criteria, in order to determine whether the model performs a task intended by a user. When the evaluation criteria according to the selection indicators are not satisfied in the performance evaluation, the processor 110 may re-adjust the training conditions and parameters of the trained artificial intelligence model and then perform additional training or may train a new model. When the evaluation criteria according to the selection indicators are satisfied in the performance evaluation, the processor 110 may select the corresponding model as an artificial intelligence model that performs a task intended by a user. In this case, satisfying the evaluation criteria according to the selection indicators in the performance evaluation may be understood as an evaluation result being equal to or higher than a preset threshold. The threshold may be adjusted by the user, or may be dynamically adjusted according to the task intended by the user.

According to the above description, an operation for the construction of an artificial intelligence model performed by the processor 110 of the present disclosure may reflect evaluation criteria customized to the user's intention in the process of training and selecting the model. Therefore, this operation may minimize the energy wasted while performing the task of adjusting training conditions and parameters according to evaluation results, and may efficiently use the resources required to develop a model that satisfies the evaluation criteria that may change each time. In other words, the present disclosure may provide an environment in which a model optimized for the purpose and domain of use can be stably developed based on such resource efficiency.

The memory 120 according to one embodiment of the present disclosure may be understood as a constituent unit including hardware and/or software for storing and managing data that is processed in the computing device 100. That is, the memory 120 may store any type of data generated or determined by the processor 110 and any type of data received by the network unit 130. For example, the memory 120 may include at least one type of storage medium of a flash memory type, hard disk type, multimedia card micro type, and card type memory, random access memory (RAM), static random access memory (SRAM), read-only memory (ROM), electrically erasable programmable read-only memory (EEPROM), programmable read-only memory (PROM), magnetic memory, a magnetic disk, and an optical disk. Furthermore, the memory 120 may include a database system that controls and manages data in a predetermined system. Since the types of memory 120 described above are only examples, the type of memory 120 may be configured in various manners within a range understandable to those skilled in the art based on the content of the present disclosure.

The memory 120 may structure, organize and manage data required for the processor 110 to perform operations, combinations of the data, and program codes executable by the processor 110. For example, the memory 120 may store the medical data received via the network unit 130 to be described later. The memory 120 may store program codes that operate an artificial intelligence model to receive medical data and perform training, program codes that operate a neural network model to receive electrocardiogram data and perform inference according to the purpose of use of the computing device 100, and processed data that is generated as the program codes are executed.

The network unit 130 according to one embodiment of the present disclosure may be understood as a constituent unit that transmits and receives data through any type of known wired/wireless communication system. For example, the network unit 130 may perform data transmission and reception using a wired/wireless communication system such as a local area network (LAN), a wideband code division multiple access (WCDMA) network, a long term evolution (LTE) network, the wireless broadband Internet (WiBro), a 5th generation mobile communication (5G) network, a ultra wide-band wireless communication network, a ZigBee network, a radio frequency (RF) communication network, a wireless LAN, a wireless fidelity network, a near field communication (NFC) network, or a Bluetooth network. Since the above-described communication systems are only examples, the wired/wireless communication system for the data transmission and reception of the network unit 130 may be applied in various manners other than the above-described examples.

The network unit 130 may receive data required for the processor 110 to perform operations through wired or wireless communication with any system, any client, or the like. Furthermore, the network unit 130 may transmit the data, generated through the operation of the processor 110, through wired or wireless communication with any system, any client, or the like. For example, the network unit 130 may receive bio-data through communication with a database within a hospital environment, a cloud server performing tasks such as the standardization of medical data, a client such as a smart watch, or a medical computing device. The network unit 130 may transmit output data of a neural network model, intermediate data and processed data obtained in the operation process of the processor 110, etc. through communication with the above-described database, server, client, or computing device.

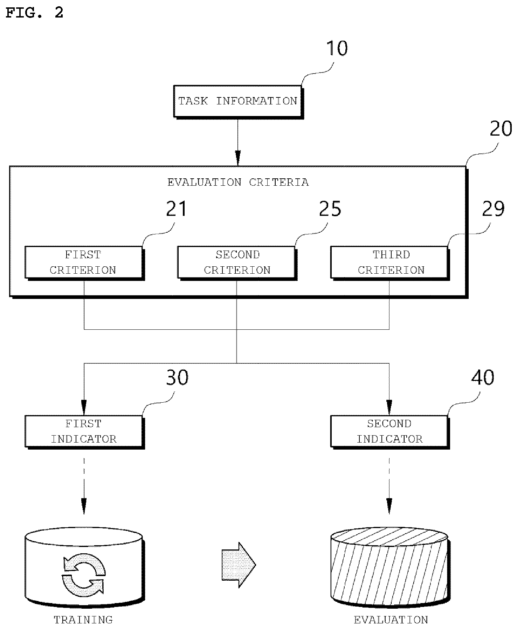

FIG. 2 is a block diagram showing an operation process for constructing a medical artificial intelligence model according to one embodiment of the present disclosure.

Referring to FIG. 2, the computing device 100 according to the embodiment of the present disclosure may establish evaluation criteria 20 for an artificial intelligence model based on task information 10 that reflects the purpose and domain of use therein. The computing device 100 may obtain the task information 10 based on user input. The task information 10 may be basic information indicating the task that an artificial intelligence model will perform for a specific disease, or may be information about evaluation criteria themselves based on the basic information. The computing device 100 may generate the evaluation criteria 20 for determining whether the artificial intelligence model can achieve appropriate performance for the purpose and domain of use by analyzing the obtained task information 10. When the task information 10 includes basic information, the computing device 100 may derive evaluation criteria 20 through keyword matching analysis, artificial intelligence-based analysis, and/or the like for the basic information. When the task information 10 includes information about evaluation criteria themselves, the computing device 100 may determine the evaluation criteria 20 according to the task information 10.

The evaluation criteria 20 established by the computing device 100 may include at least one of a first criterion 21 for the accuracy of the artificial intelligence model, a second criterion 25 for the uncertainty of the output of the artificial intelligence model, and a third criterion 29 for the correlation between the output of the artificial intelligence model and a biometric value that determines whether a disease included in a task intended by a user has occurred. The first criterion 21 may be a criterion for determining how accurately the artificial intelligence model performed a training task. For example, indicators for evaluating the first criterion 21 may include AUROC, F1 score, precision, sensitivity, recall, and/or the like. The second criterion 25 may be a criterion indicating how uncertain the result of the task performed by the artificial intelligence model is. The second criterion 25 may be a criterion for evaluating whether 60 out of 100 people actually developed a specific disease when the artificial intelligence determines that the probability of developing the specific disease is 60% based on 100 people. For example, indicators for evaluating the second criterion 25 may include adaptive calibration error (ACE) and expected calibration error (ECE). For each disease, there is a continuous value that can be measured from the body to check whether a disease has occurred. The third criterion 29 may be a criterion for determining how much a biometric value that determines whether a disease included in a task has occurred is related to the result of a task performed by the artificial intelligence model. For example, when the task intended by the user is to predict left ventricular systolic dysfunction, the third criterion 29 may be a criterion for calculating the correlation between the left ventricular ejection fraction (EF), which is a biometric value that can determine whether left ventricular systolic dysfunction has occurred, and the result value predicted by an artificial intelligence model for left ventricular systolic dysfunction. When the task intended by the user is to predict myocardial infarction, the third criterion 29 may be a criterion for calculating the correlation between the troponin level, which is a biometric value that can determine whether myocardial infarction has occurred, and the result value predicted by an artificial intelligence model for myocardial infarction.

Meanwhile, there may be a plurality of biometric values that determine whether a disease has occurred. For example, not only troponin but also creatinine kinase may be used for the biometric values to determine whether myocardial infarction has occurred. Accordingly, there may be one or more biometric values that are used as the third criterion 29.

The computing device 100 may construct the evaluation criteria 20 to include at least one of the first criterion 21, the second criterion 25, and the third criterion 29 according to the task information 10. For example, the computing device 100 may determine the ratio among the first criterion 21, the second criterion 25, and the third criterion 29 in the evaluation criteria 20 by analyzing the task information 10. When the task information 10 includes a prediction of left ventricular systolic dysfunction, the computing device 100 may determine the ratio among the first criterion 21, the second criterion 25, and the third criterion 29 in the evaluation criteria 20 to be 4:3:3. In this case, the computing device 100 may determine the ratio based on user input, or may determine the ratio through database rule-based analysis, artificial intelligence-based analysis, and/or the like.

Referring to FIG. 2, the computing device 100 may construct a first indicator 30 used for loss computation for training the artificial intelligence model and a second indicator 40 used for evaluation computation for selecting the trained model according to the established evaluation criteria 20. When the ratio among the first criterion 21, the second criterion 25, and the third criterion 29 is determined, the computing device 100 may determine a loss function included in the first indicator so that the correlation according to the third criterion 29 can be calculated. Since the third criterion 29 is a criterion specific to the purpose and domain of use, the computing device 100 may construct the loss function so that a term for calculating the third criterion 29 is included in the loss function corresponding to the first indicator 30 so that the artificial intelligence model can desirably satisfy the third criterion 29. For example, when the task information 10 includes a prediction of left ventricular systolic dysfunction, the biometric value that determines whether the disease of the third criterion 29 has occurred is determined to be the left ventricular ejection fraction so that the computing device 100 can construct a left ventricular ejection fraction regression loss function in the loss function included in the first indicator. When there are a plurality of biometric values that determine whether the disease of the third criterion 29 has occurred, the computing device 100 may generate the loss function included in the first indicator by constructing and combining loss functions corresponding to the plurality of biometric values, respectively.

In addition, when the ratio among the first criterion 21, the second criterion 25, and the third criterion 29 is determined, the computing device 100 may determine the detailed indicators included in the second indicator 40 and the ratio between the detailed indicators in the second indicator 40. The detailed indicators included in the second indicator 40 may be constructed according to the evaluation criteria. For example, when the task information 10 includes a prediction of left ventricular systolic dysfunction, the computing device 100 may construct a detailed indicator of the second indicator 40 for evaluating the first criterion 21 as a combination of F1 score, AUROC, and AUPRC (area under the precision recall curve). Furthermore, the computing device 100 may construct a detailed indicator of the second indicator 40 for evaluating the second criterion 25 as a combination of ACE and ECE.

The form of the combination may be represented by Equation 1 below:

[Equation 1]

$$\text{Detailed indicator for evaluating first criterion} = (\text{F1 score} + \text{AUROC} + \text{AUPRC})/3$$

$$\text{Detailed indicator for evaluating second criterion} = ((1 - \text{ACE}) + (1 - \text{ECE}))/2$$

The computing device 100 may select the left ventricular ejection fraction regression loss function as a detailed indicator for evaluating the third criterion 29. In the case where there are a plurality of biometric values that determine whether the disease of the third criterion 29 has occurred, the computing device 100 may construct a detailed indicator for evaluating the third criterion 29 as a combination of the plurality of biometric values. In this case, the combination may be a simple summation of detailed indicators corresponding to the plurality of biometric values, respectively, or may be the generation of a single integrated detailed indicator based on a predetermined mathematical formula. The computing device 10 may construct a detailed indicator of the second indicator 40 as described above through database rule-based analysis, artificial intelligence-based analysis, and/or the like. Furthermore, the computing device 100 may configure the ratio among the detailed indicators of the second indicator 40 configured for each of the evaluation criteria according to the ratio among the first criterion 21, the second criterion 25, and the third criterion 28.

The computing device 100 may use the first indicator 30 in the computational process in which the artificial intelligence model learns a task intended by the user. In other words, the computing device 100 may train the artificial intelligence model to perform the task intended by the user by using the first indicator 30 in the loss computation. The computing device 100 may use the second indicator 40 in the evaluation computation in which the performance of the trained model is evaluated. In other words, the computing device 100 may evaluate whether the artificial intelligence model performed the task intended by the user according to the evaluation criteria 20 by using the second indicator 40. When the performance of the artificial intelligence model evaluated using the second indicator 40 satisfies the evaluation criteria 20, the computing device 100 may select the model, satisfying the evaluation criteria 20, as the artificial intelligence model that performs the task intended by the user. When the performance of the artificial intelligence model evaluated using the second indicator 40 does not satisfy the evaluation criterion 20, the computing device 100 may additionally train the model that does not satisfy the evaluation criteria, may train a new model, or may reconstruct the evaluation criteria 200 or the first indicator 30 and the second indicator 40. In this case, whether the evaluation criteria 20 are satisfied may be determined based on whether an evaluation value calculated via the second indicator 40 is equal to or larger than a preset threshold value. For example, when the evaluation value calculated via the second indicator 40 is equal to or larger than the preset threshold value, the computing device 100 may determine that the performance of the artificial intelligence model satisfies the evaluation criteria 20. When the evaluation value calculated via the second indicator 40 is smaller than the preset threshold value, the computing device 100 may determine that the performance of the artificial intelligence model does not satisfy the evaluation criterion 20. Meanwhile, the threshold value may be adjusted by user input, or may be dynamically adjusted in compliance with set rules according to the type of detailed indicator included in the second indicator 40.

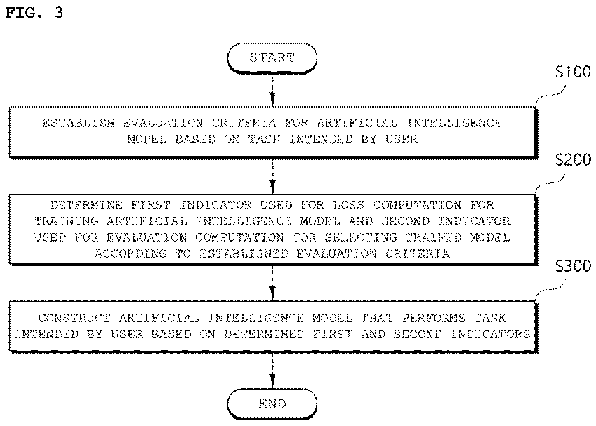

FIG. 3 is a flowchart showing a method of constructing a medical artificial intelligence model according to one embodiment of the present disclosure.

Referring to FIG. 3, the computing device 100 according to one embodiment of the present disclosure may establish evaluation criteria for an artificial intelligence model based on a task intended by a user in step S100. The computing device 100 may obtain information about the task intended by the user based on user input. Furthermore, the computing device 100 may generate the evaluation criteria by analyzing the information about the task. For example, when the computing device 100 is a client in a cloud system, the computing device 100 may receive user input via a user interface implemented via an input/output unit and generate information about a task intended by a user. When the computing device 100 is a server, the computing device 100 may receive user input through wired/wireless communication with a client and generate information about a task intended by a user. The computing device 100 may match the information about the task intended by the user with the evaluation criteria via a preset database and then store and manage them. Accordingly, the computing device 100 may identify evaluation classifications and detailed criteria corresponding to the information about the task intended by the user in the preset database. The computing device 100 may determine the identified evaluation classifications and detailed criteria as the evaluation criteria on its own, or may determine the identified evaluation classifications and detailed criteria as the evaluation criteria based on a selection according to the user input. Alternatively, the computing device 100 may derive the evaluation criteria by inputting the information about the task intended by the user to a pre-trained criteria setting model. In this case, the criteria setting model may be a pre-trained neural network model based on data labeled with evaluation criteria that match the task intended by the user.

The computing device 100 may determine a first indicator used for loss calculation for the training of the artificial intelligence model and a second indicator used for evaluation calculation for the selection of the trained model according to the evaluation criteria, established through step S100, in step S200. The computing device 10 may determine a loss function included in the first indicator in order to calculate the correlation according to a third criterion for the correlation between the output of the artificial intelligence model and a biometric value that determines whether a disease included in the task intended by the user has occurred, out of the evaluation criteria. The computing device 100 may determine detailed indicators included in the second indicator according to the ratio among the first criterion for the accuracy of the artificial intelligence model, the second criterion for the uncertainty of the output of the artificial intelligence model, and the third criterion in the evaluation criteria.

The computing device 100 may construct an artificial intelligence model that performs the task intended by the user based on the first and second indicators, determined through step S200, in step S300. The computing device 100 may train the artificial intelligence model using the loss function included in the first indicator. Furthermore, the computing device 100 may evaluate the performance of the model, trained using the loss function included in the first indicator, by using the detailed indicators included in the second indicator. When the performance of the trained model matches the task intended by the first user, the computing device 100 may select the model as a model that performs the task intended by the user.

The various embodiments of the present disclosure described above may be combined with one or more additional embodiments, and may be changed within the range understandable to those skilled in the art in light of the above detailed description. The embodiments of the present disclosure should be understood as illustrative but not restrictive in all respects. For example, individual components described as unitary may be implemented in a distributed manner, and similarly, the components described as distributed may also be implemented in a combined form. Accordingly, all changes or modifications derived from the meanings and scopes of the claims of the present disclosure and their equivalents should be construed as being included in the scope of the present disclosure.

## Claims

1. A method of constructing a medical artificial intelligence model, the method being performed by a computing device including at least one processor, the method comprising:

   establishing evaluation criteria for an artificial intelligence model based on a task intended by a user;
   determining a first indicator used for loss computation for training an artificial intelligence model and a second indicator used for evaluation computation for selecting the trained model according to the established evaluation criteria; and
   constructing an artificial intelligence model that performs the task intended by the user based on the determined first and second indicators.

2. The method of claim 1, wherein the evaluation criteria comprise at least one of:

   a first criterion for accuracy of the artificial intelligence model;
   a second criterion for uncertainty of output of the artificial intelligence model; and
   a third criterion for a correlation between the output of the artificial intelligence model and a biometric value that determines whether a disease included in the task intended by the user has occurred.

3. The method of claim 2, wherein, when the task intended by the user is a prediction of left ventricular systolic dysfunction (LVSD), the biometric value that determines whether the disease has occurred is a left ventricular ejection fraction (EF).

4. The method of claim 2, wherein establishing the evaluation criteria for the artificial intelligence model based on the task intended by the user comprises determining a ratio among the first criterion, the second criterion, and the third criterion in the evaluation criteria based on the task intended by the user.

5. The method of claim 4, wherein, when the task intended by the user is a prediction of left ventricular systolic dysfunction (LVSD), the ratio among the first criterion, the second criterion, and the third criterion in the evaluation criteria is determined to be 4:3:3.

6. The method of claim 2, wherein determining the first indicator used for the loss computation for training the artificial intelligence model and the second indicator used for the evaluation computation for selecting the trained model according to the established evaluation criteria comprises determining a loss function included in the first indicator so that a correlation according to the third criterion can be calculated.

7. The method of claim 6, wherein, when the task intended by the user is a prediction of left ventricular systolic dysfunction (LVSD), the loss function included in the first indicator includes a left ventricular ejection fraction (EF) regression loss function.

8. The method of claim 2, wherein determining the first indicator used for the loss computation for training the artificial intelligence model and the second indicator used for the evaluation computation for selecting the trained model according to the established evaluation criteria comprises determining detailed indicators included in the second indicator according to the ratio among the first criterion, the second criterion, and the third criterion in the evaluation criteria.

9. The method of claim 1, wherein establishing the evaluation criteria for the artificial intelligence model based on the task intended by the user comprises:

obtaining information about the task intended by the user based on user input; and
deriving the evaluation criteria by inputting the information about the task intended by the user to a pre-trained criteria setting model.

10. The method of claim 1, wherein establishing the evaluation criteria for the artificial intelligence model based on the task intended by the user comprises:

obtaining information about the task intended by the user based on user input; and
identifying evaluation classifications and detailed criteria corresponding to the information about the task intended by the user from a preset database.

11. The method of claim 1, wherein constructing the artificial intelligence model that performs the task intended by the user based on the determined first and second indicators comprises:

training the artificial intelligence model using the determined first indicator;
evaluating performance of the artificial intelligence model using the determined second indicator; and
when the evaluated performance of the artificial intelligence model satisfies the evaluation criteria, selecting an artificial intelligence model satisfying the evaluation criteria the an artificial intelligence model that performs the task intended by the user.

12. A computer program stored in a computer-readable storage medium, the computer program performing operations for constructing a medical artificial intelligence model when executed on one or more processors, wherein the operations comprise the operations of:

establishing evaluation criteria for an artificial intelligence model based on a task intended by a user;
determining a first indicator used for loss computation for training an artificial intelligence model and a second indicator used for evaluation computation for selecting the trained model according to the established evaluation criteria; and
constructing an artificial intelligence model that performs the task intended by the user based on the determined first and second indicators.

13. A computing device for constructing a medical artificial intelligence model, the computing device comprising:

a processor including at least one core; and
memory including program codes executable on the processor;
wherein the processor:

establishes evaluation criteria for an artificial intelligence model based on a task intended by a user;
determines a first indicator used for loss computation for training an artificial intelligence model and a second indicator used for evaluation computation for selecting the trained model according to the established evaluation criteria; and

constructs an artificial intelligence model that performs the task intended by the user based on the determined first and second indicators.

FIG. 1

<u>100</u>

PROCESSOR ~ 110

MEMORY ~ 120

NETWORK UNIT ~ 130

FIG. 2

FIG. 3

```
                          ┌─────────┐
                          │  START  │
                          └─────────┘
                               │
                               ▼
  ┌──────────────────────────────────────────────────────────────┐   S100
  │   ESTABLISH EVALUATION CRITERIA FOR ARTIFICIAL INTELLIGENCE   │
  │         MODEL BASED ON TASK INTENDED BY USER                  │
  └──────────────────────────────────────────────────────────────┘
                               │
                               ▼
  ┌──────────────────────────────────────────────────────────────┐   S200
  │    DETERMINE FIRST INDICATOR USED FOR LOSS COMPUTATION FOR    │
  │   TRAINING ARTIFICIAL INTELLIGENCE MODEL AND SECOND INDICATOR │
  │   USED FOR EVALUATION COMPUTATION FOR SELECTING TRAINED MODEL │
  │        ACCORDING TO ESTABLISHED EVALUATION CRITERIA           │
  └──────────────────────────────────────────────────────────────┘
                               │
                               ▼
  ┌──────────────────────────────────────────────────────────────┐   S300
  │   CONSTRUCT ARTIFICIAL INTELLIGENCE MODEL THAT PERFORMS TASK  │
  │  INTENDED BY USER BASED ON DETERMINED FIRST AND SECOND INDICATORS │
  └──────────────────────────────────────────────────────────────┘
                               │
                               ▼
                          ┌─────────┐
                          │   END   │
                          └─────────┘
```

## INTERNATIONAL SEARCH REPORT

International application No.

**PCT/KR2023/014431**

### A. CLASSIFICATION OF SUBJECT MATTER

**G06N 3/08**(2006.01)i; **G06N 3/04**(2006.01)i

According to International Patent Classification (IPC) or to both national classification and IPC

### B. FIELDS SEARCHED

Minimum documentation searched (classification system followed by classification symbols)

G06N 3/08(2006.01); A61B 5/02(2006.01); A61B 5/0472(2006.01); G06N 20/20(2019.01); G06N 3/02(2006.01); G06N 3/04(2006.01); G06V 10/774(2022.01); G06V 10/776(2022.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 인공지능 모델(artificial intelligence model), 의료(medical), 평가 기준(evaluation standard), 손실(loss), 좌심실 수축 기능 부전(left ventricular systolic dysfunction)

### C. DOCUMENTS CONSIDERED TO BE RELEVANT

| Category* | Citation of document, with indication, where appropriate, of the relevant passages | Relevant to claim No. |
|---|---|---|
| Y | KR 10-2022-0113881 A (BEIJING BAIDU NETCOM SCIENCE TECHNOLOGY CO., LTD.) 17 August 2022 (2022-08-17) <br> See paragraphs [0032]-[0049], [0076] and [0121]. | 1-13 |
| Y | US 2020-0397313 A1 (MAYO FOUNDATION FOR MEDICAL EDUCATION AND RESEARCH) 24 December 2020 (2020-12-24) <br> See paragraphs [0015], [0096], [0099] and [0102]. | 1-13 |
| Y | KR 10-2242516 B1 (DEEPMIND TECHNOLOGIES LIMITED) 20 April 2021 (2021-04-20) <br> See paragraph [0034]. | 4-5,8 |
| Y | KR 10-2340046 B1 (ALGORITHMLABS. LTD. et al.) 20 December 2021 (2021-12-20) <br> See paragraph [0015]; and claim 1. | 9-10 |
| A | WO 2022-008630 A1 (KONINKLIJKE PHILIPS N.V.) 13 January 2022 (2022-01-13) <br> See claim 1. | 1-13 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **04 January 2024** | **04 January 2024** |

| Name and mailing address of the ISA/KR | Authorized officer |
|---|---|
| **Korean Intellectual Property Office** <br> **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

INTERNATIONAL SEARCH REPORT
Information on patent family members

International application No.

**PCT/KR2023/014431**

| Patent document cited in search report | | | Publication date (day/month/year) | Patent family member(s) | | | Publication date (day/month/year) |
|---|---|---|---|---|---|---|---|
| KR | 10-2022-0113881 | A | 17 August 2022 | CN | 113657465 | A | 16 November 2021 |
| | | | | JP | 2022-141957 | A | 29 September 2022 |
| | | | | US | 2022-0335711 | A1 | 20 October 2022 |
| US | 2020-0397313 | A1 | 24 December 2020 | AU | 2018-346412 | A1 | 30 April 2020 |
| | | | | CA | 3078519 | A1 | 11 April 2019 |
| | | | | CN | 111432720 | A | 17 July 2020 |
| | | | | EP | 3691524 | A1 | 12 August 2020 |
| | | | | EP | 3691524 | A4 | 11 August 2021 |
| | | | | JP | 2020-536629 | A | 17 December 2020 |
| | | | | JP | 2023-089112 | A | 27 June 2023 |
| | | | | JP | 7262452 | B2 | 21 April 2023 |
| | | | | US | 2023-0089991 | A1 | 23 March 2023 |
| | | | | WO | 2019-070978 | A1 | 11 April 2019 |
| KR | 10-2242516 | B1 | 20 April 2021 | CN | 109690576 | A | 26 April 2019 |
| | | | | EP | 3485432 | A1 | 22 May 2019 |
| | | | | EP | 3485432 | B1 | 31 May 2023 |
| | | | | EP | 4231197 | A1 | 23 August 2023 |
| | | | | JP | 2019-525329 | A | 05 September 2019 |
| | | | | JP | 6824382 | B2 | 03 February 2021 |
| | | | | US | 2019-0236482 | A1 | 01 August 2019 |
| | | | | WO | 2018-017546 | A1 | 25 January 2018 |
| KR | 10-2340046 | B1 | 20 December 2021 | None | | | |
| WO | 2022-008630 | A1 | 13 January 2022 | CN | 115803751 | A | 14 March 2023 |
| | | | | EP | 3937084 | A1 | 12 January 2022 |
| | | | | EP | 4179467 | A1 | 17 May 2023 |
| | | | | JP | 2023-533188 | A | 02 August 2023 |
| | | | | US | 2023-0252305 | A1 | 10 August 2023 |

Form PCT/ISA/210 (patent family annex) (July 2022)